# EUROPÄISCHE PATENTSCHRIFT

(11) **EP 0 821 007 B1**
(45) Veröffentlichungstag und Bekanntmachung des Hinweises auf die Patenterteilung: **03.03.2004**
(21) Anmeldenummer: 97111980.5
(22) Anmeldetag: 14.07.1997
(51) Int. Cl.: C12N 15/15, C07K 14/81, A61K 38/57

(54) **Aprotinin-Variante mit verbesserten Eigenschaften**
Aprotinin variant with improved properties
Variant de l'aprotinine ayant des propriétés améliorées

(30) Priorität: 25.07.1996 DE 19629982
(43) Veröffentlichungstag der Anmeldung: 28.01.1998
(73) Patentinhaber: Schröder, Werner, Dr., 42113 Wuppertal (DE)
(72) Erfinder: Schröder, Werner, Dr., 42113 Wuppertal (DE); Bjorn, Soren, 2800 Lyngby (DK); Norris, Kjeld, 2900 Hellerup (DK); Diness, Viggo, 2900 Charlottenlund (DK); Norkskov-Lauritsen, Leif, 4733 Tappernoje (DK); Christensen, Niels Dyhr, 2300 Kopenhagen S, (DK)
(74) Vertreter: VOSSIUS & PARTNER

(56) Entgegenhaltungen:
- EP-A- 0 132 732
- EP-A- 0 339 942
- EP-A- 0 419 878
- WO-A-92/06111
- US-A- 5 621 074

## Beschreibung

Die vorliegende Erfindung betrifft Aprotinin-Varianten mit verbesserten enzyminhibitorischen, immunologischen und pharmakokinetischen Eigenschaften und deren Herstellung.

Aprotinin, das auch als boviner pankreatischer Trypsin-Inhibitor (BPTI) bezeichnet wird, gehört zur Familie der Serinproteasen-Inhibitoren vom Kunitz-Typ. Das Spektrum der inhibierbaren Serinproteasen umfaßt z.B. Trypsin, Chymotrypsin, Plasmin und Plasma-Kallikrein (W. Gebhard, H. Tschesche und H. Fritz, Proteinase Inhibitors, Barrett and Salvesen (eds.), Elsevier Science Publ. BV 375-387, 1986).

Aprotinin besteht aus 58 Aminosäuren. Die dreidimensoniale Struktur des Proteins wurde mit Hilfe von Röntgenstrukturanalyse und NMR-Spektroskopie aufgeklärt (Wlodawer et al., J. Mol. Biol. 198 (3), 469-480, 1987; Wagner et al., J. Mol. Biol. 196 (1), 227-231, 1987; Berndt et al., Biochemistry 32 (17), 4564-4570, 1993).

Unter dem Handelsnamen Trasylol® wird natürliches Aprotinin ursprünglich für die Behandlung von Pankreatitis eingesetzt. Trasylol® wird heute in der Herzchirugie verwendet, nachdem klinische Studien gezeigt haben, daß eine Behandlung mit Aprotinin den Transfusionsbedarf bei derartigen Operationen signifikant verringert und zur Reduktion von Nachblutungen führt (D. Royston, J. Cardiothorac. Vasc. Anesth. 6; 76-100, 1992).

Es konnte gezeigt werden, daß der Austausch der die Hemmspezifität festlegenden Aminosäure in Position 15 zu wertvollen Aprotinin Varianten mit verbesserten Inbihitoreigenschaften führt (DE-PS 3 339 693). Je nach der eingeführten Aminosäure können auf diese Weise potente Inihibitoren erzeugt werden, die z B. die Elastase aus Pankreas oder aus Leukozyten hemmen.

Ferner ließ sich zeigen, daß die Hemmeigenschaften von Aprotinin und der durch Austausch in der Position 15 erzeugten Varianten auch durch weitere Aminosäurereste in der Kontaktregion zwischen der zu hemmenden Zielprotease und dem Inhibitormolekül bestimmt wird. Dazu gehören vor allem die zusätzlichen Aminosäurereste in den Positionen 14, 16, 17, 18, 19, 34, 38 und 39 Aprotininvarianten mit verbesserten Eigenschaften durch Austausch einer oder mehrerer dieser Aminosäurereste im Bereich der Kontaktregion wurden u.a. in folgenden Patentanmeldungen beispielhaft beschrieben: WO 89/01968, WO 89/10374, EP 0 307 592, EP 683 229.

Interessanterweise konnten die pharmakokinetischen Eigenschaften von Aprotinin und seinen Varianten verbessert werden durch Aminosäureaustausche, die die physikochemischen Eigenschaften der Substanz festlegen. So gelang es, durch Senkung der positiven Nettoladung des Moleküls die Nierenbindung signifikant zu senken. Solche Varianten wurden in der Patentanmeldung WO 92/06111 beschrieben.

Aus Gründen der besseren technischen Herstellbarkeit ist es günstig, in bestimmten Fällen, eine Modifikation am N-terminalen Ende des Inhibitormoleküls vorzunehmen. Solche Modifikationen können N-terminale Verkürzungen oder Verlängerungen oder Deletionen von einer oder mehreren Aminosäuren sein. N-terminal modifizierte Aprotininvarianten wurden in der EP 419 878 beschrieben.

### Erfindungsgemäße Aprotininvarianten

Die in der vorliegenden Patentanmeldung beschriebenen Aprotininvarianten zeichnen sich durch folgende Merkmale aus:
1. Austausch von einer oder mehreren Aminosäuren im aktiven Zentrum des Moleküls zur Verbesserung der Aktivitätseigenschaften.
2. Austausch von Aminosäuren zur Senkung der positiven Nettoladung mit dem Ziel, die immunologischen und pharmakokinetischen Eigenschaften zu verbessern.
3. Modifikation der N-terminalen Aminosäuresequenz aus Gründen der technischen Herstellbarkeit.

Aprotininvarianten, die jeweils nur eines der genannten Merkmale enthalten, wurden in den oben zitierten Patentanmeldungen beschrieben. Die erfindungsgemäßen Aprotininvarianten vereinigen in ihrer Molekülstruktur nun zwei oder drei der oben genannten Merkmale. Die Aminosäuresequenz ist beispielhaft für einige Varianten in der Abbildung dargestellt

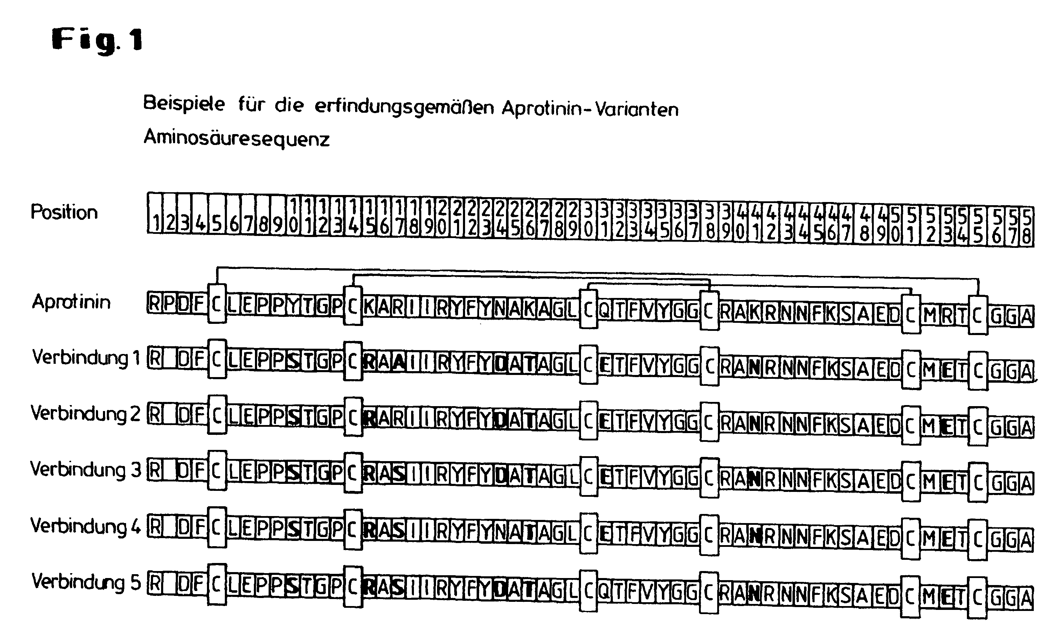

Die erfindungsgemäßen Aprotinin-Varianten beschränken sich jedoch nicht auf die in der Abbildung 1 genannten Beispiele. Zu den erfindungsgemäßen Aprotinin-Varianten gehören auch Varianten mit der N-terminalen Verlängerung Ala(-2)-Gln(-1), mit dem natürlichen Aminosäurerest Prolin in Position 2, mit dem Austausch anderer Aminosäuren, die eine positive Ladung tragen gegen neutrale oder negativ geladene Aminosäurereste, oder mit dem Austausch anderer neutraler Aminosäuren gegen negativ geladene Aminosäurereste. Die Auswahl der Austausche von Aminosäurereste folgt dabei dem Prinzip, eine Substanz zu erzeugen, die bei physiologischem pH-Wert eine reduzierte positive Nettoladung besitzt, bevorzugt im Bereich von +2 bis -2. Die genannten Änderungen in der Aminosäuresequenz einschließlich der N-terminalen Verlängerung oder Verkürzungen oder Deletionen können in beliebiger Kombination miteinander genutzt werden. Zu den erfindungsgemäßen Aprotinin-Varianten gehören somit alle Verbindungen, die eine Kombination der oben genannten Merkmale aufweisen und eine, bei physiologischem pH-Wert reduzierte positive Nettoladung besitzen.

Überraschenderweise zeigte sich, daß die Kombination von zwei oder drei der genannten Merkmale nicht nur zum Erhalt, sondern teilweise sogar zur Verstärkung der Ausprägung der Einzelmerkmale führt. Darüberhinaus konnten signifikante neue Substanzeigenschaften erzeugt werden, die sich z.B. auf die immunologischen, die pharmakokinetischen und die Oberflächenbindungseigenschaften beziehen. Die neuen Varianten zeigen eine verringerte Reaktion mit polyklonalen humanen und Kaninchen-Antiseren, die unter Verwendung von Aprotinin erzeugt wurden. Weiter wurde gefunden, daß die neuen Varianten ein deutlich verringertes immunogenes Verhalten im Vergleich zum Aprotinin besitzen, d.h. sie lösen eine verringerte Immunresponse aus. Weiterhin konnte gezeigt werden, daß die erfindungsgemäßen Varianten im Vergleich zu Aprotinin eine geringere Histaminfreisetzung aus Blutzellen induzieren. Weiter zeigen die neuen Varianten eine deutlich geringere renale Akkumulation im Vergleich zu Aprotinin. Die enzymkinetischen Hemmkonstanten (Ki-Werte) sind überraschenderweise trotz der großen Zahl an Veränderungen im Molekülkörper gegenüber früheren Varianten des Moleküls verbessert worden.

Die vorliegende Erfindung betrifft also Aprotininvarianten mit einer Nettoladung von +3 bis -3 bei pH 7 und mit den Aminosäuren Arg 15 oder Arg 15-Ala 17 Bevorzugt sind solche mit einer Nettoladung von +2 bis -2, besonders bevorzugt solche mit +1 bis -1.

Diese Aprotininvarianten eignen sich zur Hemmung von Plasmakallikrein, Gewebekallikrein und Plasmin.

Zusätzlich können diese Aprotininvarianten eine veränderte N-terminale Sequenz besitzen.

Damit sind Aprotininvarianten mit einer N-terminalen Verlängerung oder Verkürzung oder mit deletierten Aminosäuren im N-Terminus gemeint.

Bevorzugt ist die Aprotininvariante DesPro2-Ser10-Arg15-Ala17-Asp24-Thr26-Glu31-Asn41-Glu53-Aprotinin.

Die Erfindung betrifft auch Arzneimittel enthaltend diese Aprotininvarianten.

Der beschriebene neuartige Proteinaseninhibitor eignet sich für die Behandlung von Krankheitszuständen, bei denen es - auch infolge komplexer chirurgischer Verfahren wie z.B. in der Herzchirurgie oder beim alloarthroplastischen Gelenkersatz in der Transplantationsmedizin - zur Aktivierung der plasmatischen Enzymsysteme durch ausgedehnten oder intensiven Fremdoberflächenkontakt des Blutes kommt.

Der Inhibitor reduzierte den Blutverlust bei Operationen, die mit einem erhöhten Blutungsrisiko (z.B. Herzoperationen, Knochen- und Gelenkchirurgie) verbunden sind. Sie eignen sich zur Therapie bei Schock, Polytrauma und Schädel- und Hirntrauma, Sepsis, disseminiert intravasale Koagulopathie (DIC), Multi-Organversagen, entzündlichen Erkrankungen mit Beteiligung des Kallikreinsystems wie rheumatische Gelenkerkrankungen und Asthma. Er verhindert das invasive Tumorwachstum und die Metastasierung durch Hemmung von Plasmin. Er eignet sich auch zur Schmerz- und Ödemtherapie durch Hemmung der Bradykininsynthese sowie zur Behandlung des Schlaganfalles.

Er ist auch brauchbar bei der Dialysetherapie und künstlichen Organen, um Entzündungen und Koagulation zu verhindern und um das Risiko von Blutungen zu vermindern.

### Herstellung der erfindungsgemäßen Aprotinin-Varianten

Für die Herstellung der erfindungsgemäßen Aprotinin-Variante ist es günstig, gentechnische Verfahren einzusetzen. Hierzu wird mit gängigen molekularbiologischen Methoden in einen geeigneten mikrobiellen Expressionsorganismus die gentechnische Information für die Synthese der jeweils betrachteten Aprotininvariante eingeführt. Der rekombinante Mikroorganismus wird fermentiert; durch Wahl geeigneter Bedingungen wird die heterologe Erbinformation zur Expression gebracht. Die exprimierte Aprotininvariante wird im Anschluß aus der Kulturbrühe gewonnen.

Geeignete Wirtorganismen für die Produktion der erfindungsgemäßen Aprotininvarianten können Bakterien, Hefen oder Pilze sein. Die Expression kann intrazellulär oder unter Verwendung geeigneter Sekretionssysteme extrazellulär erfolgen. Die Aprotininvariante kann korrekt prozessiert oder fusioniert an Peptide oder Proteine exprimiert werden.

Geeignete Systeme zur Expression von Aprotininvarianten wurden in den Patentanmeldungen EP 683 229, WO 89/02463, WO 90/10075 und in verschiedenen weiteren der oben bereits genannten Patentanmeldungen beschrieben.

### Methoden zur Durchführung der Erfindung

### Enzyme

Die verwendeten Enzyme (Restriktionsendonucleasen, Alkalische Phosphatase aus Kälberdarm, T4 Polynucleotidkinase und T4 DNA Ligase) wurden von den Firmen Boehringer Mannheim und GIBCO/BRL bezogen und nach Vorschriften der Hersteller eingesetzt.

### Molekularbiologische Techniken

Routinemäßige Klonierungsarbeiten, wie z.B. die Isolation von Plasmid-DNA aus E. coli (sog. miniprep) und die Transformation von E. coli mit Plasmid-DNA wurden nach Sambrook et al. (Molecular Cloning Cold Spring Harbor, 1989) durchgeführt. Als Wirtorganismus für Transformationen wurde der E coli Stamm DH5α (GIBCO/BRL) eingesetzt. Zur Isolation größerer Mengen Plasmid-DNA wurden Qiagen-tips (Qiagen) verwendet. Die Extraktion von DNA-Fragenten aus Agarosegelen wurde mit Hilfe von Jetsorb nach Angaben des Herstellers (Genomed) durchgeführt.

Oligonukleotide für "site directed mutagenesis"-Experimente und Primer für PCR- und Sequenzierungs-Reaktionen wurden mit dem "380 A DNA synthesizer" der Firma Applied Biosystems hergestellt. Die Mutageneseversuche wurden nach einem Verfahren von Deng und Nickoloff durchgeführt (Deng et al., Anal. Biochem. 200, 81-88, 1992) unter Verwendung eines Kits der Firma Pharmacia Biotech ("Unique Site Elimination Mutagenesis"). Alle Vektorkonstruktionen und Mutageneseexperimente wurden durch Taq Cycle-DNA-Sequenzierung mit Fluoreszenz-markierten Terminatoren auf einem "ABI 373 A Sequencer" (Applied Biosystems) bestätigt.

### Transformation von Saccharomyces cerevisiae

Hefezellen, z.B. der Stamm JC34. 4D (MATα, ura3-52, suc2) wurden in 10 ml YEPD (2 % Glucose; 2 % Pepton; 1 % Difco Hefeextrakt) angezogen und bei einer O.D._{600 nm} von 0,16 bis 0,8 geerntet. Die Zellen wurden mit 5 ml Lösung A (1 M Sorbitol; 10 mM Bicin pH 8,35; 3 % Ethylenglycol) gewaschen, in 0,2 ml Lösung A resuspendiert und bei -70°C gelagert.

Plasmid DNA (5 µg) und Carrier DNA (50 µg DNA aus Heringssperma) wurden zu den gefrorenen Zellen gegeben. Die Zellen wurden anschließend durch Schütteln für 5 min bei 37°C aufgetaut. Nach Zugabe von 1,5 ml Lösung B (40 % PEG 1000; 200 mM Bicin pH 8,35) wurden die Zellen für 60 min bei 30°C inkubiert, mit 1,5 ml Lösung C (0,15 M NaCl; 10 mM Bicin pH 8,35) gewaschen und in 100 µl Lösung C resuspendiert. Die Ausplattierung erfolgt auf einem Selektivmedium mit 2 % Agar. Transformanden wurden nach einer Inkubation von 3 Tagen bei 30°C erhalten

### Nährmedien für die Fermentation

### 1. SD2-Medium:

| | |
|---|---|
| Bacto Yeast Nitrogen Base | 6,7 g/l |
| Glucose* | 20 g/l |
| KH₂PO₄ | 6,7 g/l |
| | pH 6,0 |

| | |
|---|---|
| * = getrennt autoklavieren | |

### 2. SC5-Medium:

| | |
|---|---|
| Glucose* | 20 g/l |
| Difco Hefeextrakt | 20 g/l |
| KH₂PO₄ | 6,7 g/l |
| (NH₄)₂SO₄ | 2,0 g/l |
| MgSO₄ x 7 H₂O | 1,0 g/l |
| Spurenelementlösung SL4 | 1,0 ml/l |
| | pH 6,0 |

| | |
|---|---|
| * = getrennt autoklavieren | |

### Spurenelementlösung SL4:

| | |
|---|---|
| Titriplex III | 5 g/l |
| FeSO₄ x 7 H₂O | 2 g/l |
| ZnSO₄ x 7 H₂O | 0,1 g/l |
| MnCl₂ x 4 H₂O | 0,03 g/l |
| H₃BO₃ | 0,3 g/l |
| CoCl₂ x 6 H₂O | 0,2 g/l |
| CuCl₂ x 2 H₂O | 0,01 g/l |
| NiCl₂ x 6 H₂O | 0,02 g/l |
| Na₂MoO₄ x 2 H₂O | 0,03 g/l |

### 3. Fermentermedium:

| | |
|---|---|
| Glucose* | 2,0 g/l |
| Sojapepton | 25,0 g/l |
| KH₂PO₄ | 1,4 g/l |
| MgSO₄ x 7 H₂O | 1,0 g/l |
| Thiaminchlorid | 5,1 mg/l |
| Inosit | 20 mg/l |
| Spurenelementlösung | 3 ml/l |
| Vitaminlösung | 3 ml/l |
| (NH₄)₂SO₄ | 3,8 g/l |
| | pH 5,5 |

| | |
|---|---|
| * = getrennt autoklavieren | |

### Futterlösung:

| | |
|---|---|
| Glucose* | 530 g/l |
| (NH₄)₂SO₄ | 5,0 g/l |
| KH₂PO₄ | 2,9 g/l |
| MgSO₄ x 7 H₂O | 3,8 g/l |
| Thiaminchlorid | 13 mg/l |
| Inosit | 70 mg/l |
| Spurenelementlösung | 6,8 ml/l |
| Vitaminlösung | 6,8 ml/l |

| | |
|---|---|
| * = getrennt autoklavieren | |

### Spurenelementlösung:

| | |
|---|---|
| FeCl₃ x 6 H₂O | 13,5 g/l |
| ZnCl₂ x 4 H₂O | 2,0 g/l |
| H₃BO₃ | 0,5 g/l |
| CoCl₂ x 6 H₂O | 2,0 g/l |
| CuSO₄ x 5 H₂O | 1,9 g/l |
| Na₂MoO₄ x 2 H₂O | 2,0 g/l |
| CaCl₂ x 2 H₂O | 1,0 g/l |
| HCl konz. | 100 ml/l |

### Vitaminlösung:

| | |
|---|---|
| Riboflavin | 0,42 g/l |
| Ca-Pantothenat | 5,9 g/l |
| Nicotinsäure | 6,1 g/l |
| Pyridoxinhydrochlorid | 1,7 g/l |
| Biotin | 0,06 g/l |
| Folsäure | 0,04 g/l |

### Herstellung von Arbeitskonserven

Mit einer Stammkonserve wurden 200 ml SD2-Medium in einem 1 l-Erlenmeyerkolben 1 %ig beimpft. Die Kultur wurde 72 Stunden bei 28°C auf den Schüttler (260 Upm) inkubiert. Danach wurden je 2 ml in Konservengefäße abgefüllt und in Flüssigstickstoff eingefroren.

### Fermentation im Schüttelkolben

Als Vorkultur wurden 200 ml SD2-Medium in einem 1 l Kolben 1 %ig mit einer Arbeitskonserve beimpft und 72 Stunden bei 28°C auf dem Schüttler (260 Upm) fermentiert. Mit der Vorkultur wurden Hauptkulturen (200 ml SC5-Medium in 1 l-Kolben) 1 %ig beimpft und 72-96 Stunden auf dem Schüttler bei 28°C inkubiert.

### Fermentation in 10 l-Bioreaktoren

Als Vorkultur wurden 200 ml SD2-Medium in einem 1 l Kolben 1 %ig mit einer Arbeitskonserve beimpft und 72 Stunden bei 28°C auf dem Schüttler (260 Upm) fermentiert. Die Hauptkultur im 10 l-Fermenter wurde als fed-batch Fermentation über 96 Stunden durchgeführt. Als Nährmedium wurde Fermentermedium verwendet, das Startvolumen betrug 7 l. Der Fermenter wurde mit 200 ml Vorkultur beimpft

### Fermentationsbedingungen:

Temperatur 28°C
Rührerdrehzahl: 500 Upm
Belüftung: 10 l/min
pH: 5,5
Kopfraumdruck: 200 mbar
Nach 7 Stunden Fermentationszeit wurde die Fütterung gestartet. Die Fütterrate wurde über den Respiratorischen Quotienten (RQ) gesteuert (RQ = gebildetes CO₂ / verbrauchter Sauerstoff). Stieg der RQ auf Werte > 1,15 an, wurde die Fütterrate verringert, sank er auf Werte < 1,05 wurde die Fütterrate erhöht.

In regelmäßigen Abständen wurden Proben aus dem Fermenter entnommen und das Zellwachstum durch Messung der optischen Dichte bei 700 nm bestimmt. Außerdem wurde die Konzentration von Bay 19-8757 im Überstand durch Aktivitätsmessung ermittelt.

Bei Fermentationsende wurde der pH-Wert durch Zugabe von 50 % (w/v) Zitronensäure auf 3,0 abgesenkt und der Fermenter für 10 min auf 70°C erhitzt. Die Zellen wurden anschließend durch Zentrifugation bei 7 500 x g abgetrennt und der Überstand zur Proteinreinigung abgegeben.

### Materialien für die proteinchemische Analytik

Die Sequenzanalysen wurden mit einem Proteinsequenzer Model 473A der Firma Applied Biosystems (Forster City, USA) durchgeführt. Das Standardsequenzierungsprogramm wurde verwendet. Der Sequenzer, die verschiedenen Sequenzierungsprogramme sowie das PTH-Detektionssystem sind im Detail im Bedienungshandbuch beschrieben (User's manual protein sequencing system model 473 A) (1989) Applied Biosystems Forster City, CA 94404, USA).

Die Reagenzien für den Betrieb des Sequenzers und die HPLC-Säule für die PTH-Detektion wurden von Applied Biosystems bezogen.

Die HPLC-Analysen wurden mit einem HP 1090 HPLC-System von Hewlett Packard (D-Waldbronn) durchgeführt. Eine RP-18-HPLC-Säule (250 mm x 4,6 mm, 5 µ-Material, 300 Angström Porendurchmesser) von Backerbond (D-Groß Gerau) wurde für die Trennung verwendet.

Die Kapillarelektrophorese Modell 270 A-HT war von Applied Biosystems (Forster City, CA 94404, USA). Die Proben wurden in der Regel hydrodynamisch über verschiedene Zeitintervalle injiziert. Die verwendete Kapillarsäule (50 µm x 72 cm) war von Applied Biosystems.

Die Aminosäureanalysen wurden mit einem Aminosäureanalysator LC 3000 von Eppendorf Biotronik (D-Maintal) durchgeführt. Ein leicht modifiziertes Standardtrennprogramm von Biotronik wurde benutzt Die Trennprogramme und die Funktion des Analysators sind ausführlich im Handbuch des Gerätes beschrieben.

Die Molekulargewichte wurden mit einem MALDI I System von Kratos/Shimadzu (D-Duisburg) bestimmt Die SDS-Elektrophoresen wurden durchgeführt mit einem Elektrophoresesystem von Pharmacia (D-Freiburg).

Die Bestimmung der kinetischen Daten wurde mit dem Mikrotiterplattenreader von SLT (D-Crailsheim) durchgeführt. Das Waschen der Mikrotiterplatten wurde mit einem Waschgerät von Dynatec (D-Denkendorf) durchgeführt.

Enzyme und Substrate waren von Calbiochem (D-Bad Soden). Alle anderen Chemikalien und Reagenzien waren von Merck (D-Darmstadt) oder Sigma (D-Deisenhofen). 96 Well Platten wurden von Greiner bezogen.

Die polyklonalen Kaninchen anti-Aprotininantikörper wurden im Kaninchen durch Immunisierung mit Aprotinin erzeugt. Die humanen polyklonalen anti-Aprotininantikörper stammen von Patienten, die mit Aprotinin behandelt wurden.

### Proteinchemische Analytik

### N-terminale Sequenzanalyse

1-3 nmol in Wasser gelöster Proteaseinhibitor wurden auf ein Sequenzersheet, das mit Polybren vorinkubiert war, geladen. Das Protein wurde mit dem fast normal Sequenzerzyklus sequenziert. Die PTH-Aminosäuren wurden mittel Online-HPLC mit Hilfe eines 50 pmol PTH-Standards idenifiziert.

### Aminosäureanalyse

200 µg Protein wurden in 200 µl 6N HCl gelöst und 1h bei 166°C hydrolysiert. Ca. 1 nmol der Probe wurde auf den Aminosäureanalysator gegeben. Der Menge an Aminosäure wurde über einen 5 nmol Standard ermittelt.

### SDS-Gelelektrophorese

Die SDS-Gelelektrophorese wurde nach den Bedingungen von Laemmli durchgeführt. 10 µg des Proteaseinhibitors wurden mit einem 10-20 %igem SDS-Gel analysiert und mittels Silberfärbung (Merril et al.) sichtbar gemacht.

U.K. Laemmli, Nature 227, 680-685 (1970)
C.R. Merril, M.L. Dunau, D. Goldmann, Anal. Biochem. 100. 201-207 (1981).

### Kapillarelektrophorese

8 ng des Proteaseinhibitors wurden mittels Kapillarelektrophorese an einer Glassäule (Länge 72 cm, Innendurchmesser 50 µm) untersucht. Bedingungen: Stromstärke 90 µA, Säulentemperatur 25°C, 100 nM Phosphatpuffer pH 3,0, Detektion 210 nm, Aufgabe unter Druck 3 Sek.

### Reverse Phase Chromatographie

5 nmol des Proteaseinhibitors wurden an einer Bakerbond RP-18-HPLC-Säule (5 µ-Material, 4,6 nm x 250 mm, 300 Angström Porengröße) chromatographiert. Als Eluent wurde ein Acetonitril/TFA-Gradient verwendet. Bedingungen: Fluß 0,7 ml/min, Säulentemperatur 40°C, Detektion 40°C, Lösungsmittel A, 0,1 % TFA, Lösungsmittel B 0,1 % TFA / 60 % Acetonitril; Gradient: 0 min. 0 % B, 10 min 0 % B, 70 min. 100 % B, 80 min 0 % B.

### Molekulargewichtsbestimmung

1 µg des Proteaseinhibitors wurde mit der MALDI-Technik anakysiert. Als Matrix wurde Sinapinsäure verwendet. Die Standardproteine für die Massenkalibrierung waren Rinderinsulin, Cytochrom C und Melittin.

### Proteingehalt

Der Proteingehalt wird bestimmt nach der BCA-Methode. Bei dieser Methode werden durch die anwesenden Proteine Cu²⁺-Ionen zu Cu¹⁺-Ionen umgesetzt, die mit Bicinchoninsäure einen Komplex bilden, der bei 560 nm absorbiert.

Das lyophilisierte Protein wird ins Feuchtegleichgewicht gebracht und mit einer Konzentration von 1 mg/ml in 0,9 % NaCl-Lösung gelöst Es wird eine Verdünnungsreihe hergestellt. Zu 50 µl Probenlösung werden 1000 µl BCA-Testreagenz (Pierce) gegeben, das Reagenzröhrchen mit einem Stopfen fest verschlossen und exakt 30 Minuten bei 60 °C inkubiert. Nach Abkühlen der Proben im Eisbad für 5 Minuten erfolgt die Messung bei einer Temperatur von 25°C und einer Wellenlänge von 560 nm.

### Aktivität (Trypsin-Hemmtest, titrimetrisch)

Die Aktivität wird bestimmt nach einem modifizierten F.I.P.-Trypsin-Hemmtest. Bay y 19-8757 hemmt die Trypsin-katalysierte Hydrolyse von Nα-benzoyl-L-argininethylester (BAEE). Die bei der Reaktion freiwerdene Carboxylgruppen werden durch die Alkali-Titration ermittelt. Die Trypsin-Restaktivität ist ein Maß für die inhibitorische Aktivität des Wirkstoffs.

Das lyophilisierte Protein wird ins Feuchtegleichgewicht gebracht, mit einer Konzentration von 1 mg/ml in 0,9 % NaCl-Lösung gelöst und eine Verdünnungsreihe hergestellt. Zu 1 ml Probenlösung werden 2 ml Puffer (15 mM Boratpuffer, pH 8,0 mit 200 mM CaCl₂) und 0,8 ml Trypsinlösung (2 mg/ml) gegeben und 5 Minuten bei 25°C inkubiert. Anschließend wird 0,2 ml BAEE-Lösung (6,8 mg/ml) hinzugegeben und der KOH-Verbrauch über 5 Minuten gemessen.

### Bestimmung der Kreuzreaktion der Proteaseinhibitoren mit polyklonalen Kaninchen bzw. humanen Anti-Aprotininantikörpern

0,5 - 10 ng Proteaseinhibitor bzw. Aprotinin gelöst im Kupplungspuffer wurden über Nacht bei 4°C an eine Mikrotiterplatte gebunden. Die Wells wurden 4 mal mit je 200 µL Waschpuffer gewaschen und dann 100 µL der Blockierungslösung zugesetzt. Die Platte wurde abgedeckt und eine Stunde bei 37°C inkubiert. Nach dem Waschen wie oben beschrieben wurden der polyklonale Kaninchen Anti-Aprotininantikörper (0,2 µg/ml in 1 % BSA in PBS-Puffer) bzw. der polyklonale humane Antikörper (20 µg/ml in 1 % HSA in PBS-Puffer) hinzugegeben. Die Platte wurde abgedeckt und eine Stunde bei 37°C inkubiert und dann gewaschen wie oben beschrieben. Nun wurden 100 µL biotinylierter anti-Kaninchen bzw. anti-human Antikörper (25 µL + 10 ml 1 % BSA bzw 1 % HSA in PBS-Puffer) hinzugegeben und eine Stunde bei 37°C inkubiert. Die Platte wurde gewaschen wie oben beschrieben und dann 100 µL Streptavidin-Peroxidasekomplex (50 µL + 10 ml 1 % BSA bzw 1 % HSA in PBS-Puffer) zu jedem Well gegeben. Die Platte wurde abgedeckt und eine Stunde bei 37°C inkubiert und dann wie oben beschrieben gewaschen.

Die Substratreaktion wurde mit TMB-Substrat + Peroxidaselösung (1+1; 100 µL pro Well) durchgeführt. Die Reaktion wurde nach 10 min mit 100 µL/Well 2 M Phosphorsäure gestoppt und die Absorption bei 450 nm gemessen (Referenz 570 nm).

### Lösungen:

| | |
|---|---|
| 1. Inkubationspuffer | 15 nM Na₂CO₃, 35 mM NaHCO₃, pH 9,6 |
| 2. Probenpuffern | Die Proben wurden in einer geeigneten Konzentration im Inkubationspuffer gelöst. |
| 3. Waschlösung | 0,1 % (v/v) Tween-20 in PBS |
| 4. Blockierungspuffer | 3 % (w/v) BSA bzw HSA in PBS. |

### Beispiele

### Beispiel 1

### Herstellung eines Hefe-Expressionsvektors zur Sekretion von rekombinantem DesPro2-Ser10-Arg15-Ala17-Asp24-Thr26-Glu31-Asn41-Glu53-Aprotinin

Als Ausgangsmaterial für die Herstellung des DesPro2-Ser10-Arg15-Ala17-Asp24-Thr26-Glu31-Asn41-Glu53-Aprotinin Gens diente ein DesPro2-Arg15-Ala17 Gen, das über die Restriktionsenzyme HindIII und BamHI in den Vektor pUC18 kloniert wurde. Der resultierende Vektor (pEM6.6.L) wurde mit dem Mutagenese Primer A und dem Scal/Mlul U.S E. Selektionsprimer einer Doppelstrang-Mutagenese-Reaktion nach dem U.S.E.-Verfahren (Pharmacia Biotech) unterzogen.. Der Mutagenese Primer A hatte folgende Sequenz:

### Primer A

5'GGCTGCAGAGCTAACCGTAACAACTTCAAATCCGCGGAAGACTGCATG GAAACTTGCGGTGGTGCTTAG 3'. Dieser Primer generiert die Mutationen Asn41 und Glu53 im DesPro2-Arg15-Ala17 Aprotinin Gen. Die Analyse der Klone erfolgte durch einen Restriktionsverdau mit den Enzymen Scal und SphI. Die gewünschte Sequenz wurde außerdem durch DNA-Sequenzierung des Klons pEM31.8.L bestätigt. Die weiteren Austausche im 5'-Bereich des Gens (Ser10-Asp24-Thr26-Glu31) wurden mit Hilfe der PCR-Technik unter Verwendung des Primers B und des 'reversen 24-mer M13' Primers ausgehend von pEM31.8.L Plasmid DNA erzeugt. Der Primer B hatte folgende Sequenz:

### Primer B

5'TGCCTCGAGCCGCCGTCTACTGGGCCCTGCAGAGCTATCATCCGTTACT TCTACGATGCAACTGCAGGCCTGTGTGAAACCTTCGTATACGGC 3'. Die Xhol Erkennungssequenz ist unterstrichen.

Der PCR-Ansatz enthielt 20 ng pEM31.8.L Plasmid DNA, 20 pmol 'reversen 24-mer M13' Primer, 60 pmol Primer B, 200 µM dNTPs, 1 x PCR Reaktionspuffer II (Perkin Elmer), 4 mM MgCl₂ und 2,5 U Taq DNA Polymerase (Perkin Elmer) in einem Gesamtvolumen von 100 µl. Die 'Cycle'-Bedingungen waren 3 min bei 94°C, 30 Zyklen mit jeweils 1 min bei 94°C, 1 min bei 55°C und 1 min bei 72°C und eine anschließende 5 min Inkubation bei 72°C. Der PCR-Ansatz wurde 1:5 verdünnt und mit dem Vektor pCRII (Invitrogen) ligiert. Mit dem Ligationsansatz wurden E. coli DH5α Zellen transformiert. Positive Klone wurden nach einem Restriktionsverdau mit den Enzymen XhoI und BamHI identifiziert und mehrere Klone sequenziert. Der Klon pES9.10.L enthielt die gewünschte Sequenz und wurde für die weiteren Arbeiten eingesetzt.

Ein E.coli/Hefe Schaukelvektor (z.B. pA202) wurde für die Konstruktion eines Hefe-Sekretionsvektors verwendet, in dem die DesPro2-Ser10-Arg15-Ala17-Asp24-Thr26-Glu31-Asn41-Glu53-Aprotinin-Sequenz mit der Hefe Alpha-Faktor pre-pro-Sequenz verknüpft ist.

Der Vektor pA202 trägt ein Ampicillin Resistenzgen (bla) und ein URA3 Gen als selektierbare Markergene für E.coli und Hefe. Weitere essentielle Elemente des Vektors sind der Co1 E1 und der 2 µ Ursprungspunkt der Replikation (ori). Der REP3 Locus befindet sich ebenfalls in dieser Region. Ein 1200 Bp großes EcoRI-HindIII-Fragment trägt den MFα1 Promotor und die N-terminale pre-pro-Sequenz des Hefe Alpha-Faktor-Vorläuferproteins (Kurjan und Herskowitz, Cell 30, 933 943, 1982). Durch Einführung einer modifizierten DesPro2-Arg-15-Aprotinin cDNA als HindIII-BamHI Fragment wurde die Erkennungsstelle für die KexII-Protease ('Lys-Arg') innerhalb der Alpha-Faktor-pre-pro-Sequenz wieder hergestellt (EP 0 419 878).

Am 3'-Ende der DesPro2-Arg-15-Aprotinin Sequenz trägt der Vektor ein BamHI-SaII Fragment des Hefe URA3 Gens, das in dieser Position als Terminationssignal für die Transkription fungiert (Yarger et al., Mol. Cell. Biol. 6, 1095-1101, 1986).

Ein 180 Bp großes DNA-Fragment wurde mit Xhol und BamHI aus dem Vektor pES9.10.L herausgeschnitten, über Agarosegel-Elektrophorese gereinigt und in den ebenfalls mit Xhol und BamHI geschnitten und dephosphorylierten Vektor pA202 kloniert. Durch diese Klonierung wird das DesPro2-Arg15-Aprotinin im Vektor pA202 durch das DesPro2-Ser10-Arg15-Ala17-Asp24-Thr26-Glu31-Asn41-Glu53-Aprotinin ersetzt Hefezellen (JC34.4D) wurden mit dem aus dieser Klonierung resultierenden Vektor pES13.10.L transformiert.

Andere E Coli/Hefe Schaukelvektoren mit unterschiedlichen Promotoren, wie z.B. der konstititive GAPDH oder der induzierbare GAL 10 Promotor, können in ähnlicher Weise hergestellt werden und führen ebenfalls zur Sekretion des DesPro2-Ser10-Arg15-Ala17-Asp24-Thr26-Glu31-Asn41-Glu53-Aprotinins.

Daneben ist es selbstverständlich auch möglich, Schaukelvektoren mit anderen Hefe-Replikationsursprüngen, wie z.B. das chromosomal autonom replizierende Segment (ars), einzusetzen.

Geeignete selektierbare Markergene sind neben dem URA3 Gen solche Gene, die einer auxotrophen Hefemutante zur Prototrophie verhelfen, wie z.B. die LEU2, HIS3 oder TRP1 Gene. Außerdem können selbstverständlich auch Gene eingesetzt werden, deren Produkte Resistenz gegenüber verschiedenen Antibiotika, wie z.B. dem Aminoglykosid G418, vermitteln.

Andere Hefen, wie z.B. die methylotrophen Hefen Pichia pastoris oder Hansenula polymorpha, sind nach Transformation mit geeigneten Vektoren ebenfalls in der Lage, DesPro2-Ser10-Arg15-Ala17-Asp24-Thr26-Glu31-Asn41-Glu53-Aprotinin zu produzieren.

### Beispiel 2

### Herstellung eines Hefe-Expressionsvektors zur Sekretion von rekombinantem Ser10-Arg15-Ala17-Asp24-Thr26-Glu31-Asn41-Glu53-Aprotinin mit der natürlichen N-terminalen Sequenz 'Arg-Pro-Asp'

Zur Herstellung eines Hefe-Expressionsvektors, der die Sekretion von Ser10-Arg15-Ala17-Asp24-Thr26-Glu31-Asn41-Glu53-Aprotinin mit der natürlichen N-terminalen Sequenz 'Arg-Pro-Asp' erlaubt, wurde zunächst der MFα1 Promotor mit der α-Faktor pre-Sequenz und dem 5'Ende des Aprotinin Gens (bis zur Erkennungssequenz des Restriktionsenzyms Xhol) über PCR amplifiziert und kloniert. Die verwendeten Primer hatten folgende Sequenz:

### Primer C

5'GGGATATCTATTGATAAGATTTAAAGGTATTTGACAAG 3' Die EcoRV Erkennungssequenz ist unterstrichen.

### Primer D.

5'GGGCTCGAGGCAGAAATCTGGTCTAGCCAAAGCAGAAGAAGCAGCGAA CAAGACAGCAGTGAAAATAGATGGAATCTCATTCTTTTAATCGTTTATATT 3'. Die Xhol Erkennungssequenz ist unterstrichen.

Der PCR-Ansatz enthielt 200 ng pA202 Plasmid DNA, 0,2 µM Primer C, 0,2 µM Primer D, 200 µM dNTPs, 1 x PCR Reaktionspuffer 11 (Stratagene, Opti-Prime™) und 2,5 U Taq DNA Polymerase (Perkin Elmer) in einem Gesamtvolumen von 50 µl. Die 'Cycle'-Bedingungen waren: 1 min bei 94°C, 30 Zyklen mit jeweils 1 min bei 94°C, 1 min bei 50°C und 2 min bei 72°C und eine anschließende 5 min Inkubation bei 72°C. Der PCR-Ansatz wurde 1:5 verdünnt und mit dem Vektor pCRII (Invitrogen) ligiert. Mit dem Ligationsansatz wurden E. coli DH5α Zellen transformiert. Positive Klone wurden nach einem Restriktionsverdau mit dem Enzym EcoRI identifiziert und mehrere Klone sequenziert. Der Klon pIU20.11.L wurde für die weiteren Arbeiten eingesetzt.

Der E coli/Hefe Schaukelvektor pYES2 (Invitrogen) wurde für die Konstruktion eines Hefe-Sektretionsvektors verwendet, in dem die Ser10-Arg15-Ala17-Asp24-Thr26-Glu31-Asn41-Glu53-Aprotinin-Sequenz direkt mit der Hefe Alpha-Faktor pre-Sequenz verknüpft ist. Zunächst wurde der Vektor pYES2 mit den Restriktionsenzymen SspI und BamHI geschnitten, dephosphoryliert und gelgereinigt. Der auf dem Vektor pYES2 vorhandene GAL1 Promotor und der fl ori werden hierbei entfernt. Ein ca. 1030 Bp großes DNA Fragment wurde mit EcoRV und Xhol aus dem Vektor pIU20.11.L herausgeschnitten, über Agarosegel-Elektrophorese gereinigt und zusammen mit einem ca. 180 Bp großen Xhol und BamHI Fragment aus dem Vektor pES9.10.L in den mit SspI und BamHI geschnittenen Vektor pYES2 kloniert. Mit dem Ligationsansatz wurden E. coli DH5α Zellen transformiert. Positive Klone wurden nach einem Restriktionsverdau mit dem Enzym Xhol identifiziert und sequenziert. Hefezellen (JC34.4D) wurden mit dem aus dieser Klonierung resultierenden Vektor pIU28.11.L transformiert. Der Expressionsvektor pIU28.11.L enthält keine α-Faktor pro-Sequenz mehr, so daß die Prozessierung des Ser10-Arg15-Ala17-Asp24-Thr26-Glu31-Asn41-Glu53-Aprotinins ausschließlich durch die Signalpeptidase erfolgt und unabhängig wird von der Spaltung durch die KexII Protease.

### Beispiel 3

### Fermentation von Saccharomyces cerevisiae

Ein Saccharomyces cerevisiae Expressionsstamm wurde wie oben beschrieben fermentiert.

### Beispiel 4

### Reinigung von Aprotinin-Derivaten ohne Nettoladung bei neutralem pH-Wert

### 1. Übersicht über geeignete Reinigungsverfahren

Nach der Fermentation werden die Zellen durch Zentrifugation abgetrennt und der verbleibende Überstand wird filtriert, um zurückgebliebene Zellen zu entfernen.

Der zellfreie Überstand wird durch Zusatz von konzentrierter Zitronensäure auf pH 3 eingestellt. Die Lösung muß mit gereinigtem Wasser geeignet verdünnt werden, um eine Leitfähigkeit von weniger als 8 mS/cm einzustellen. Im Anschluß wird die Lösung auf eine Kationenaustauscher-Säule aufgetragen, die vorher mit einem sauren Puffer äquilibriert worden ist. Nicht gebundenes Material wird durch ausgiebiges Waschen mit Startpuffer entfernt. Das Produkt wird mit Hilfe eines Salzgradienten eluiert. Die erhaltenen Fraktionen werden mit Hilfe von Umkehrphasen-Hochdruckflüssigkeitschromatographie (RP-HPLC) und einem biologischen Aktivitätstest, der die Protease-Inhibition bestimmt, auf ihren Produktgehalt hin untersucht. Fraktionen, die das Produkt enthalten, werden vereinigt und direkt auf eine präparative RP-HPLC Säule aufgetragen. Die Säule wurde vorher mit einem sauren Puffer äquilibriert. Nicht gebundenes Protein wird durch Waschen der Säule mit Startpuffer entfernt. Das Produkt wird mit Hilfe eines organischen Lösungsmittel-Gradienten eluiert. Die Fraktionen werden wie bereits oben beschrieben erneut auf den Produktgehalt hin untersucht und diejenigen Fraktionen, die Produkt enthalten, werden vereinigt. In Abhängigkeit von der erreichten Reinheit des Produktes ist es möglicherweise notwendig die vereinigten Fraktionen über eine zweite RP-HPLC Säule zu reinigen. Die Bedingungen sind im wesentlichen die gleichen wie bereits beschrieben. Die erhaltene Produktlösung wird mit Wasser für Injektionszwecke verdünnt und in geeigneten Portionen abgefüllt und gefriergetrocknet.

Andere Methoden für die Reinigung von Aprotinin-Derivaten ohne Nettoladung bei neutralem pH-Wert, die mit den oben beschriebenen Prozessen kombiniert werden können, sind Affinitätschromatographie an Sepharose-immobilisiertem Trypsin und Gelpermeationschromatographie.

### 2. Reinigung von DesPro2-Ser10-Arg15-Ala17-Asp24-Thr26-Glu31-Asn41-Glu53-Aprotinin

Material aus 10 L-Fermentationen wurde nach dem folgenden Verfahren gereinigt. Nachdem die Fermentation abgeschlossen war, wurde der Fermenterinhalt mit konzentrierter Zitronensäure auf pH 3 eingestellt und für 10 Minuten auf 70°C erhitzt. Anschließend wurden die Zellen durch Zentrifugation (15 Minuten, 7500 x g , Heraeus Zentrifuge) entfernt und der erhaltene Überstand filtriert (8 µm bis 0,2 µm, Millipore, Deutschland). Aus dieser Stufe kann der Überstand durch Einfrieren bei -18°C bis zur weiteren Verwendung aufbewahrt werden. Die Lösung wurde anschließend durch Zusatz von gereinigtem Wasser auf eine Leitfähigkeit von weniger als 8 mS/cm verdünnt und auf eine SP-Sepharose FF Säule (Pharmacia, Schweden) aufgetragen. Die Säule war vorher mit 50 nM Zitrat-NaOH Puffer, pH 3 äquilibriert worden. Nicht gebundenes Protein wurde durch intensives Waschen mit dem gleichen Puffer entfernt. Anschließend wurde das Produkt mit Hilfe eines Salzgradienten (1 M NaCl) eluiert. Die erhaltenen Fraktionen wurden mit Hilfe von Umkehrphasen-Hochdruckflüssigkeitschromatographie (RP-HPLC, C4) und durch Testen der Protease-Inhibitionsaktivität auf den Produktgehalt hin untersucht. Diejenigen Fraktionen die das gewünschte Produkt enthalten wurden vereinigt.

Anschließend wurde die Produktlösung direkt auf die erste RP-HPLC Säule (Source 15 RPC, Pharmacia, Schweden) aufgetragen, die vorher mit 0,1 % Trifluoressigsäure/Wasser äquilibriert worden war. Nicht gebundenes Protein wurde durch intensives Waschen mit dem gleichen Puffer entfernt Das Produkt wurde mit Hilfe eines linearen Azetonitril-Gradienten (0-70 %) eluiert. Die erhaltenen Fraktionen wurden erneut auf den Produktgehalt hin mit den oben beschriebenen Methoden untersucht und diejenigen die Produkt enthielten wurden vereinigt.

Für die abschließende Reinigung wurde die produkthaltige Lösung mit Wasser für Injektionszwecke verdünnt und auf die zweite RP-HPLC Säule (Vydac C8, Vydac, USA) aufgetragen, die vorher mit 0,1 % Trifluoressigsäure/Wasser äquilibriert worden war, Nicht gebundenes Protein wurde durch intensives Waschen mit dem gleichen Puffer entfernt. Das Produkt wurde mit Hilfe eines linearen Azetonitril-Gradienten (0-70 %) eluiert. Die erhaltenen Fraktionen wurden erneut auf den Produktgehalt hin mit den oben beschriebenen Methoden untersucht und diejenigen, die Produkt enthielten, wurden vereinigt.

Die erhaltene Produktlösung wurde mit Wasser für Injektionszwecke verdünnt, in geeigneten Portionen abgefüllt (20, 10, 1 und 0,2 mg), lyophilisiert und analysiert.

### Beispiel 5

### Bestimmung des Ki-Wertes von humanen Plasmakallikrein mit DesPro-Ser10-Arg15-Ala17-Asp24-Thr26-Glu31-Asn41-Glu53-Aprotinin

### Beispiel:

1 Unit humanes Plasmakallikrein wurde auf 16 ml mit Puffer verdünnt (0,05 M Tris-/0,1 M NaCl, 0,05 % Tween-20; pH 8,2). 200 µL dieser Enzymlösung wurden mit absteigenden Volumina Testpuffer versetzt (250, 240, 230, 220, 200, 180, 170, 150, 100, 50 µL) und dann steigende Mengen Inhibitor im Assaypuffer hinzugesetzt (10, 20, 30, 50, 70, 80, 100, 150, 200 und 250 µL; Konzentration 0,7 µg/µL).

Die Enzym/Inhibitor-Lösung wurde 4 Stunden bei Raumtemperatur vorinkubiert. Dann 180 µL von jeder Lösung in das Well einer Microtiterplatte gegeben und mit 20 µL Substratlösung versetzt. Die Änderung der Absorption wurde bei 405 nm für 10 min gemessen. Die Geschwindigkeit der Enzymreaktionen wurde bestimmt und der Ki-Wert daraus berechnet nach der Methode von Bieth (Biochemical Medicine 32: 387-97 (1984)).

| | |
|---|---|
| Substrat Stock Lösung | 0,1 M in DMSO |
| Substrat Lösung: | 1 x 10⁻³M S-2302 in Assay-Puffer |
| Assay Puffer: | 0,05 M Tris-(hydroxymethyl)-aminomethan, 0,1M NaCl, 0,05 % Tween-20; pH 8,2; 1 mL Benzylalkohol / L. |

Die kinetischen Konstanten der Komplexierung mit den Enzymen Plasmin Faktor XIa, Rindertrypsin und Chymotrypsin wurden nach der gleichen Verfahrensweise bestimmt. Die Substrate waren Chromozym PL für Plasmin, HD-Pro-Phe-Arg-pNA für Faktor XI, S-2444 für Trypsin und Suc-Phe-Leu-Phe-pNA für Chymotrypsin.

### Beispiel 6

### Ergebnisse der proteinchemischen Charakterisierung von DesPro2-Ser10-Arg15-Ala17-Asp24-Thr26-Glu31-Asn41-Glu53-Aprotinin

Der Proteaseinhibitor DesPro2-Ser10-Arg15-Ala17-Asp24-Thr26-Glu31-Asn41-Glu53-Aprotinin wurde mittels eines gentechnisch veränderten Hefeorganismus durch Sekretion hergestellt. Aus dem Hefeüberstand wurde er durch verschiedenen chromatographische Verfahren bis zur Homogenität gereinigt. Die Identität des Inhibitors mit der klonierten Sequenz zeigen die nachfolgenden proteinanalytischen Untersuchungen.

### N-terminale Sequenzanalyse

Der Proteaseinhibitor wurde vollständig über 57 Stufen sequenziert. Die folgende Liste zeigt die bestimmte Proteinsequenz, die identisch ist mit der klonierten Sequenz. 
1
Arg-Asp-Phe-Cys-Leu-Glu-Pro-Pro-Ser-Thr-Gly-Pro-Cys-Arg-Ala-Ala-Ile-Ile-Arg-Tyr-
21
Phe-Tyr-Asp-Ala-Thr-Ala-Gly-Leu-Cys-Glu-Thr-Phe-Val-Tyr-Gly-Gly-Cys-Arg-Ala-
40
Asn-Arg-Asn-Asn-Phe-Lys-Ser-Ala-Glu-Asp-Cys-Met-Glu-Thr-Cys-Gly-Gly-Ala
 Sequenzanalyse von DesPro2-Ser10-Arg15-Ala17-Asp24-Thr26-Glu31-Asn41-Glu53-Aprotinin über 57 Stufen.

### Aminosäureanalyse

Die Aminosäureanalyse stellt einen wichtigen quantitativen Parameter für die Charakterisierung eines Proteins dar. Neben dem Proteingehalt wird bei bekannter Primärstruktur die Anzahl der Einzelaminosäuren ermittelt. Die Aminosäureanalyse von DesPro2-Ser10-Arg15-Ala17-Asp24-Thr26-Glu31-Asn41-Glu53-Aprotinin ist in guter Übereinstimmung mit den theoretischen Werten aus der Primärstruktur (Tab. 1).

**Tabelle 1**

| Aminosäureanalyse von DesPro2-Ser10-Arg15-Ala17-Asp24-Thr26-Glu31-Asn41-Glu53-Aprotinin. Die ganzen Zahlen sind auf Ala=7 bezogen. | | |
|---|---|---|
| Aminosäure | ganze Zahlen | theor. Zahlen |
| CysSO₃H | 5,28 | 6 |
| Asp | 6,27 | 6 |
| Thr | 3,49 | 4 |
| Ser | 1,58 | 2 |
| Glu | 4,25 | 4 |
| Gly | 6,16 | 6 |
| Ala | 7,00 | 7 |
| Val | 0,98 | 1 |
| Met | 1,10 | 1 |
| Ile | 1.66 | 2 |
| Leu | 1,89 | 2 |
| Tyr | 2,49 | 3 |
| Phe | 4,11 | 4 |
| Lys | 1,05 | 1 |
| Arg | 4,73 | 5 |
| Pro | 3,23 | 3 |
| ∗) Cysteine und Methionin wurden nach der Perameisensäureoxidation bestimmt (Met als Methioninsulfon). | | |

### Reverse Phase Chromatographie

Bei der HPLC-Chromatographie von Proteinen an chemisch gebundene Umkehrphasen kommt es über eine hydrophoben Wechselwirkung der Proteine zu einer Bindung an die verwendete Phase. Die Proteine werden gemäß der Stärke ihrer Bindung an die stationäre Phase von organischen Lösungsmitteln (mobile Phase) verdrängt. Aus diesem Grund ist diese Methode ein gutes Kriterium für die Beurteilung der Reinheit eines Proteins. DesPro2-Ser10-Arg15-Ala17-Asp24-Thr26-Glu31-Asn41-Glu53-Aprotinin eluiert als einziger Peak von der RP-18-Phase. Es wurde gezeigt, daß der isolierte Proteaseinhibitor sehr sauber ist.

### CE-Chromatographie

Die Kapillarelektrophorese erlaubt die Trennung von Peptiden und Proteinen aufgrund ihrer Ladung im elektrischen Feld. Die Güte der Trennung hängt dabei vom Puffer, dem pH-Wert, der Temperatur und den verwendeten Additiven ab. Als Kapillaren werden sogenannte "fused silica" Säulen mit einem Innendurchmesser von 50-100 µm eingesetzt. DesPro2-Ser10-Arg15-Ala17-Asp24-Thr26-Glu31-Asn41-Glu53-Aprotinin wurde an einer "fused silica" Säile im elektrischen Feld getrennt. Das Elektropherogramm zeigt einen schlanken Peak.

### Molekulargewichtsbestimmung

Das Molekulargewicht von DesPro2-Ser10-Arg15-Ala17-Asp24-Thr26-Glu31-Asn41-Glu53-Aprotinin wurde mit der MALDI-Technik zu 6223 Dalton bestimmt Das ermittelte Molekulargewicht ist dabei in guter Übereinstimmung mit dem theoretischen Wert von 6215 Dalton im Rahmen der Genauigkeit der Messmethode. Sinapinsäure wurde als Matrix eingesetzt.

### SDS-Gelelektrophorese

DesPro2-Ser10-Arg15-Ala17-Asp24-Thr26-Glu31-Asn41-Glu53-Aprotinin wurde mittels SDS-Elektrophorese unter reduzierenden und nicht reduzierenden Bedingungen analysiert. Sie zeigt eine Bande im Bereich von ca. 6,5 kD.

### Beispiel 7

### Bestimmung der Ki-Werte für die Komplexierung von Enzymen mit DesPro2-Ser10-Arg15-Ala17-Asp24-Thr26-Glu31-Asn41-Glu53-Aprotinin

Die inhibitorischen Konstanten von DesPro2-Ser10-Arg15-Ala17-Asp24-Thr26-Glu31-Asn41-Glu53-Aprotinin wurden für verschiedene Enzyme bestimmt. In der Tabelle 2 sind die Ki-Werte wiedergegeben.

**Tabelle 2:**

| Inhibitorische Konstanten der Komplexierung von DesPro2-Ser10-Arg15-Ala17-Asp24-Thr26-Glu31-Asn41-Glu53-Aprotinin mit den Enzymen und Plasmakallikrein, Faktor XIa, Rinderchymotrypsin und Rindertrypsin. | |
|---|---|
| **Enzym** | **Ki-Wert [M]** |
| Plasmakallikrein | 2x10⁻¹¹ |
| Plasmin | 5x10⁻¹⁰ |
| Faktor XIa | 5x10⁻¹⁰ |
| Trypsin | 2x10⁻¹¹ |
| Chymotrypsin | 9x10⁻⁹ |

### Beispiel 8

### Wechselwirkung der Proteaseinhibitoren mit polyklonalen Kaninchen bzw. humanen Anti-Aprotininantikörpern

Die rekombinant hergestellten Proteaseinhibitoren wurden auf ihre Kreuzreaktivität mit polyklonalen Kaninchen bzw. humanen Anti-Aprotininantikörpern hin untersucht. Es wurde festgstellt, daß die verschiedenen Proteaseinhibitorvarianten nur eine sehr schwache Wechselwirkung mit den Aprotinin-Antiseren zeigen.

## Patentansprüche

1. Aprotininvariante DesPro2-Ser10-Arg15-Ala17-Asp24-Thr26-Glu31-Asn41-Glu53-Aprotinin.

2. Arzneimittel enthaltend die Aprotininvariante gemäß dem Anspruch 1

3. Verwendung der Aprotininvariante gemäß dem Anspruch 1 zur Herstellung von Arzneimitteln zur Anwendung in der Chirugie, zur Senkung des Blutverlustes, bei Polytrauma, Schock und Entzündung.

## Claims

1. Aprotinin variant DesPro2-Ser10-Arg15-Ala17-Asp24-Thr-26-Glu31-Asn41-Glu53-aprotinin.

2. Pharmaceutical composition containing the aprotinin variant according to claim 1.

3. Use of the aprotinin variant according to claim 1 for the preparation of pharmaceutical compositions for use in surgery, for decreasing blood loss, in the case of a polytrauma, shock or inflammation.

## Revendications

1. Variant de l'aprotinine DesPro2-Ser10-Arg15-Ala17-Asp24-Thr26-Glu31-Asn41-Glu53-Aprotinine.

2. Médicament comprenant le variant de l'aprotinine selon la revendication 1.

3. Utilisation du variant de l'aprotinine selon la revendication 1 pour la fabrication de médicaments pour utilisation en chirurgie pour diminuer de la perte de sang, lors d'un polytraumatisme, un choc et une inflammation.
